# EUROPÄISCHE PATENTSCHRIFT

(11) **EP 4 031 503 B1**
(45) Veröffentlichungstag und Bekanntmachung des Hinweises auf die Patenterteilung: **22.07.2026**
(21) Anmeldenummer: 20775616.4
(22) Anmeldetag: 18.09.2020
(51) Int. Cl.: C03C 3/097, A61K 6/807, A61K 6/816, A61K 6/818, A61K 6/822, A61K 6/824, A61K 6/833, A61K 6/838, C03C 4/00, C03C 10/00

(54) **SPINELL-GLASKERAMIK, VERFAHREN ZU DEREN HERSTELLUNG UND GEFORMTES DENTALPRODUKT ENTHALTEND DIE SPINELL-GLASKERAMIK**
SPINEL GLASS CERAMIC, METHOD FOR THE PRODUCTION THEREOF AND SHAPED DENTAL PRODUCT CONTAINING THE SPINEL GLASS CERAMIC
VITROCÉRAMIQUE À STRUCTURE SPINELLE, SON PROCÉDÉ DE PRODUCTION ET PRODUIT DENTAIRE FAÇONNÉ CONTENANT LA VITROCÉRAMIQUE À STRUCTURE SPINELLE

(30) Priorität: 19.09.2019 DE 102019214284
(43) Veröffentlichungstag der Anmeldung: 27.07.2022
(73) Patentinhaber: Fraunhofer-Gesellschaft zur Förderung der angewandten Forschung e.V., 80686 München (DE)
(72) Erfinder: DURSCHANG, Bernhard, 97082 Würzburg (DE); PROBST, Jörn, 97082 Würzburg (DE); SOMOROWSKY, Ferdinand, 97082 Würzburg (DE)
(74) Vertreter: Pfenning, Meinig & Partner mbB
(86) Internationale Anmeldenummer: PCT/EP2020/076188
(87) Internationale Veröffentlichungsnummer: WO 2021/053195

(56) Entgegenhaltungen:
- WO-A1-2014/161770
- Titel: "Der Einfluß von P2O5 auf die Struktur und Eigenschaften von Gläsern und Glaskeramiken des Systems MgO-Al2O3-SiO2" (Dissertation) Friedrich-Schiller-Universität Jena, Deutschland Autor: Axel Katzschmann XP055511553

## Beschreibung

Die vorliegende Erfindung betrifft eine Spinell-Glaskeramik aus einer Zusammensetzung mit den Komponenten 25 bis 50 Gew.-% SiO₂, 10 bis 35 Gew.-% Al₂O₃, 1 bis 15 Gew.-% MgO, 1 bis 15 Gew.-% P₂O₅, 1 bis 25 Gew.-% ZrO₂ und/oder TiO₂, 0,1 bis 20 Gew.-% La₂O₃, 0 bis 10 Gew.-% B₂O₃, sowie 0 bis 15 Gew.-% Zusatzstoffe. Die Spinell-Glaskeramik enthält mindestens eine Spinell-Phase, jedoch keine Hochquarzmischkristallphase. Die erfindungsgemäße Glaskeramik weist eine sehr hohe mechanische Stabilität, z.B. eine sehr hohe Biegebruchfestigkeit, auf, wobei sie gleichzeitig bezüglich ihrer optischen Eigenschaften einstellbar ist. Zudem betrifft die vorliegende Erfindung auch ein Verfahren zur Herstellung sowie die Verwendung der Spinell-Glaskeramik. Im Weiteren betrifft die vorliegende Erfindung ein die Spinell-Glaskeramik enthaltendes geformtes Dentalprodukt.

Hochfeste Glaskeramiken sind von vielfältigem Interesse. Insbesondere im Dentalbereich werden für größere Restaurationen, wie drei- oder mehrgliedrigere Brücken im Backenzahnbereich, hochfeste, transluzente Glaskeramiken benötigt. Auf dem Markt kommen die Glaskeramiken mit den höchsten Biegebruchwerten aus dem Lithiumsilicat-System. Solche Glaskeramiken werden beispielsweise in EP 1 688 397, US 2017/267575 oder EP 2 723 303 beschrieben und weisen maximale Biegebruchfestigkeiten von bis zu 400 MPa auf (3-Punkt-Biegebruchfestigkeit nach DIN EN ISO 6872).

Zudem sind aus dem Stand der Technik Glaskeramiken aus dem Hochquarzmischkristallsystem bekannt. Diese beispielsweise in der US 2016/0051451 A1 beschriebenen Glaskeramiken haben sich jedoch nicht durchgesetzt, da sie in der Kombination von Optik/Transluzenz und mechanischen Eigenschaften nicht mit den auf dem Markt befindlichen Lithiumsilicat-Glaskeramiken konkurrieren können.

Die WO 2014/161770 A1 betrifft Magnesiumoxid-Aluminiumoxid-Siliciumoxid-Glas oder -Glaskeramiken auf Basis des Hochquarzmischkristall-Systems, die in einer Zwischenstufe der Kristallisation einfach mechanisch bearbeitbar sind und nach der vollständigen Kristallisation eine hochfeste, hoch transluzente und chemisch stabile Glaskeramik darstellen. Die Gläser bzw. Keramiken sind dadurch gekennzeichnet, dass sie weiterhin Phosphor und eine Übergangsmetallkomponente aufweisen, ausgewählt unter Titan und Zirkonium oder einer Mischung aus beidem.

In der Dissertation "Der Einfluss von P₂O₅ auf die Struktur und Eigenschaften von Gläsern und Glaskeramiken des Systems MgO-Al₂O₃-SiO₂" von Axel Katzschmann, Friedrich-Schiller-Universität Jena, wurde der Einfluss von P₂O₅ auf die Struktur und Eigenschaften von Gläsern und Glaskeramiken des Systems MgO-Al₂O₃-SiO₂-(TiO₂) untersucht. Im Mittelpunkt der Arbeit standen dabei neben der chemisch physikalischen Charakterisierung der modifizierten Gläser und Glaskeramiken Untersuchungen zum Einfluss von P₂O₅ auf das Phasentrennungs-, Keimbildungs- und Kristallisationsverhalten der Gläser.

Für Anwendungen, insbesondere dentale Anwendungen, bei denen Biegebruchfestigkeiten von über 400 MPa notwendig sind, gibt es bisher keine brauchbare Lösung aus dem Glaskeramik-Bereich. Stattdessen werden z.B. im Dentalbereich sinterbare Nano-Zirkonoxidkeramiken eingesetzt, die jedoch insbesondere bei den optischen Eigenschaften nicht mit Glaskeramiken vergleichbar sind.

Ausgehend hiervon war es die Aufgabe der vorliegenden Erfindung, eine Glaskeramik anzugeben, die eine sehr hohe mechanische Stabilität, insbesondere eine sehr hohe Biegebruchfestigkeit, und gleichzeitig vorteilhafte optische Eigenschaften aufweist.

Diese Aufgabe wird bezüglich einer Spinell-Glaskeramik mit den Merkmalen des Patentanspruchs 1, bezüglich eines Verfahrens zur Herstellung einer Spinell-Glaskeramik mit den Merkmalen des Patentanspruchs 8 und bezüglich eines die Spinell-Glaskeramik enthaltenden geformten Dentalprodukts mit den Merkmalen des Patentanspruchs 12 gelöst. Die jeweilig abhängigen Patentansprüche stellen dabei vorteilhafte Weiterbildungen dar.

Erfindungsgemäß wird somit eine Spinell-Glaskeramik aus einer Zusammensetzung mit den folgenden Komponenten angegeben:
25 bis 50 Gew.-% SiO₂,
10 bis 35 Gew.-% Al₂O₃,
1 bis 15 Gew.-% MgO,
1 bis 15 Gew.-% P₂O₅,
1 bis 25 Gew.-% ZrO₂ und/oder TiO₂,
0,1 bis 20 Gew.-% La₂O₃,
0 bis 10 Gew.-% B₂O₃, sowie
0 bis 15 Gew.-% Zusatzstoffe,
wobei sich die Anteile der Komponenten zu 100 Gew.-% ergänzen.

Unter einer Spinell-Glaskeramik wird dabei erfindungsgemäß eine Glaskeramik verstanden, die mindestens eine Spinell-Phase (bzw. Spinell-Kristallphase) enthält.

Unter Spinell wird vorzugsweise Magnesiumspinell (z.B. mit der Zusammensetzung MgAl₂O₄) verstanden. Besonders bevorzugt ist das Spinell ein Magnesium-Aluminium-Spinell, z.B. mit der Zusammensetzung MgAl₍₂₊ₓ)O_{(4+1,5x)}, wobei x eine Zahl von 0 bis 1 ist.

Die erfindungsgemäße Spinell-Glaskeramik enthält keine Hochquarzmischkristallphase. Zudem weist die erfindungsgemäße Spinell-Glaskeramik eine Lanthanphosphat-Kristallphase auf.

Die erfindungsgemäße Glaskeramik zeichnet sich durch Ihre spezielle Zusammensetzung aus. Aufgrund dieser Zusammensetzung und der speziellen Herstellung der Glaskeramik über eine zweistufige Wärmebehandlung enthält die Glaskeramik mindestens eine Spinell-Kristallphase, jedoch keine Hochquarzmischkristallphase. Aufgrund der Anwesenheit dieser Spinell-Kristallphase weist die Glaskeramik eine sehr hohe mechanische Festigkeit auf und kann somit auch bei Anwendungen verwendet werden, die eine besonders hohe mechanische Festigkeit erfordern. Beispielsweise können mit der erfindungsgemäßen Glaskeramik bei deren Herstellung in kleinen Chargen Biegebruchfestigkeiten von über 400 MPa (gemessen nach DIN EN ISO 6872) erzielt werden. Somit weist das System ein enormes Potential für die homogene Herstellung in größeren Chargen auf, da hier erfahrungsgemäß die Festigkeit um ca. 100 MPa steigen kann.

Zudem weist die erfindungsgemäße Glaskeramik besonders vorteilhafte optische Eigenschaften auf. So kann die Glaskeramik bezüglich ihrer optischen Eigenschaften von hochtransluzent bis opak eingestellt werden. Mit anderen Worten kann die erfindungsgemäße Spinell-Glaskeramik sowohl als hochtransluzente Glaskeramik als auch als opake Glaskeramik aber auch in vielen Zwischenstufen erhalten werden.

Eine bevorzugte Ausführungsform der erfindungsgemäßen Spinell-Glaskeramik zeichnet sich dadurch aus, dass die Spinell-Glaskeramik aus einer Zusammensetzung mit folgenden Komponenten besteht:
30 bis 45 Gew.-%, bevorzugt 33,5 bis 45 Gew.-%, besonders bevorzugt 35 bis 40 Gew.-% SiO₂,
15 bis 30 Gew.-%, bevorzugt 20 bis 25 Gew.-% Al₂O₃,
3 bis 10 Gew.-%, bevorzugt 5 bis 8 Gew.-% MgO,
2 bis 10 Gew.-%, bevorzugt 3 bis 8 Gew.-% P₂O₅,
8 bis 20 Gew.-%, bevorzugt 12 bis 18 Gew.-% ZrO₂ und/oder TiO₂,
0,1 bis 15 Gew.-%, bevorzugt 8 bis 12 Gew.-% La₂O₃,
0,1 bis 5,0 Gew.-%, bevorzugt 1,0 bis 2,0 Gew.-% B₂O₃, sowie
0,1 bis 10,0 Gew.-%, bevorzugt 1,0 bis 8,0 Gew.-% Zusatzstoffe.

Vorzugsweise enthält die Zusammensetzung der Spinell-Glaskeramik 33,5 bis 50 Gew.-%, besonders bevorzugt 34,0 bis 50 Gew.-%, ganz besonders bevorzugt 34,5 bis 50 Gew.-%, insbesondere 35,0 bis 50 Gew.-%, SiO₂.

Erfindungsgemäß enthält die Zusammensetzung der Spinell-Glaskeramik sowohl 1 bis 15 Gew.-% P₂O₅ als auch 0,1 bis 20 Gew.-% La₂O₃. Die Spinell-Glaskeramik kann Spinell und Lanthanphosphat enthalten. Vorzugsweise enthält die Spinell-Glaskeramik kein Monazit.

Eine weitere bevorzugte Ausführungsform der erfindungsgemäßen Spinell-Glaskeramik ist dadurch gekennzeichnet, dass die Spinell-Glaskeramik Spinell als Hauptkristallphase aufweist. Die erfindungsgemäße Glaskeramik kann somit vorzugsweise eine Spinell-Phase als Hauptkristallphase aufweisen. Unter einer "Hauptkristallphase" ist dabei vorzugsweise die Kristallphase der Glaskeramik zu verstehen, die von allen in der Glaskeramik vorhandenen Kristallphasen den höchsten Massenanteil hat. Alternativ kann die Glaskeramik auch mehrere (z.B. zwei) Hauptkristallphasen aufweisen, wobei diese Hauptkristallphasen dann höhere Massenanteile haben als die weiteren in der Glaskeramik vorhandenen Kristallphasen. In diesem Fall kann die Glaskeramik somit neben Spinell als Hauptkristallphase z.B. noch eine zweite Hauptkristallphase aufweisen.

Vorzugsweise beträgt der Anteil der Spinell-Hauptkristallphase an allen in der Spinell-Glaskeramik vorhandenen Kristallphasen mindestens 30 Gew.-%, besonders bevorzugt mindestens 50 Gew.-%.

Neben Spinell kann die erfindungsgemäße Glaskeramik noch mehrere weitere Kristallphasen aufweisen. Die erfindungsgemäße Spinell-Glaskeramik weist eine Lanthanphosphat-Kristallphase auf. Durch die Anwesenheit sowohl einer Spinell-Phase (bzw. Spinell-Kristallphase) als auch einer Lanthanphosphat-Kristallphase in der Glaskeramik weist die Glaskeramik eine besonders hohe mechanische Festigkeit auf.

In einer weiteren bevorzugten Ausführungsform der erfindungsgemäßen Spinell-Glaskeramik sind die Zusatzstoffe ausgewählt aus der Gruppe bestehend aus Fluor, Natriumoxid, Bariumoxid, Strontiumoxid, Zinkoxid, Calciumoxid, Yttriumoxid, Nioboxid, Tantaloxid, Lanthanoxid, Keimbildnern, insbesondere Zinnoxid und/oder Edelmetalle, Fluoreszenzmitteln, Farbstoffen, Hilfsstoffen, sowie Mischungen hiervon.

Dabei ist es bevorzugt, dass die Fluoreszenzmittel ausgewählt sind aus der Gruppe bestehend aus Oxiden von Neodym, Praseodym, Samarium, Europium, Terbium, Dysprosium, Holmium, Erbium, sowie Mischungen hiervon.

Weiterhin ist es bevorzugt, dass die Farbstoffe ausgewählt sind aus der Gruppe bestehend aus
- glasfärbenden Oxiden, bevorzugt Oxiden von Eisen, Titan, Cer, Kupfer, Chrom, Cobalt, Nickel, Mangan, Selen, Silber, Indium, Gold und Oxiden von Seltenerdmetallen
- Farbkörpern, bevorzugt dotierten Spinellen, sowie
- Mischungen hiervon.

Eine weitere bevorzugte Ausführungsform der erfindungsgemäßen Spinell-Glaskeramik zeichnet sich dadurch aus, dass die Spinell-Glaskeramik eine Biegebruchfestigkeit, gemessen nach DIN EN ISO 6872, von mehr als 400 MPa, vorzugsweise von mehr als 500 MPa, aufweist.

Die vorliegende Erfindung betrifft auch ein Verfahren zur Herstellung der erfindungsgemäßen Spinell-Glaskeramik bei dem
a) ein Ausgangsglas hergestellt wird, das die Komponenten der Spinell-Glaskeramik enthält,
b) das Ausgangsglas mindestens einer ersten Wärmebehandlung und anschließend mindestens einer zweiten Wärmebehandlung unterzogen wird.

In einer bevorzugten Variante des erfindungsgemäßen Verfahrens erfolgt die mindestens eine erste Wärmebehandlung bei einer Temperatur von 700 °C bis 950 °C, vorzugsweise von 800 °C bis 900 °C, und/oder über einen Zeitraum von 15 min bis 360 min, vorzugsweise von 120 min bis 240 min.

Weiterhin ist es bevorzugt, dass die mindestens eine zweite Wärmebehandlung bei einer Temperatur von 900 °C bis 1200 °C, vorzugsweise von 950 °C bis 1000 °C, und/oder über einen Zeitraum von 5 min bis 200 min, vorzugsweise von 60 min bis 120 min, erfolgt.

Zudem betrifft die vorliegende Erfindung die Verwendung der erfindungsgemäßen Spinell-Glaskeramik als Dentalmaterial, als Komponente eines Dentalmaterials oder als hochfestes Substrat für Bauteile, insbesondere elektronische Bauteile. Beispielsweise kann die erfindungsgemäße Spinell-Glaskeramik in einer molaren Brücke eingesetzt werden.

Die vorliegende Erfindung betrifft ferner ein geformtes Dentalprodukt enthaltend eine erfindungsgemäße Spinell-Glaskeramik, insbesondere in Form eines Inlays, eines Onlays, einer Brücke, z.B. einer molaren Brücke, eines Stiftaufbaus, einer Verblendung, einer Krone oder einer Teilkrone.

Anhand der nachfolgenden Beispiele soll die vorliegende Erfindung näher erläutert werden, ohne diese auf die hier gezeigten spezifischen Ausführungsformen und Parameter zu beschränken.

### Ausführungsbeispiele

In einem ersten Ausführungsbeispiel wird zunächst ein Ausgangsglas mit folgender Zusammensetzung hergestellt:
38,25 Gew.-% SiO₂,
22,15 Gew.-% Al₂O₃,
6,51 Gew.-% MgO,
7,06 Gew.-% P₂O₅,
7,66 Gew.-% ZrO₂,
4,97 Gew.-% TiO₂,
11,75 Gew.-% La₂O₃,
1,64 Gew.-% B₂O₃.

Das Ausgangsglas wird einer ersten Wärmbehandlung unterzogen, welche bei einer Temperatur von 900 °C und über einen Zeitraum von 2 Stunden erfolgt, und anschließend einer zweiten Wärmebehandlung unterzogen, welche bei einer Temperatur von 1000 °C und über einer Zeitraum von 2 Stunden erfolgt. Es wird eine erfindungsgemäße Spinell-Glaskeramik erhalten, die eine maximale Biegebruchfestigkeit, gemessen nach DIN EN ISO 6872, von 546 MPa aufweist. Durch Untersuchung der hergestellten Glaskeramik mittels Röntgendiffraktometrie und TEM wurde festgestellt, dass diese eine Spinell-Phase (bzw. Spinell-Kristallphase) und eine Lanthanphosphat-Kristallphase aber keine Hochquarzmischkristallphase enthält.

In einem zweiten Ausführungsbeispiel wird zunächst ein Ausgangsglas mit folgender Zusammensetzung hergestellt:
35,01 Gew.-% SiO₂,
23,80 Gew.-% Al₂O₃,
5,23 Gew.-% MgO,
3,26 Gew.-% P₂O₅,
8,83 Gew.-% ZrO₂,
6,76 Gew.-% TiO₂,
11,24 Gew.-% La₂O₃,
1,57 Gew.-% B₂O₃,
1,80 Gew.-% CeO₂,
2,00 Gew.-% Y₂O₃,
0,50 Gew.-% Tb₅O₁₁.

Das Ausgangsglas wird einer ersten Wärmebehandlung unterzogen, welche bei einer Temperatur von 800 °C und über einen Zeitraum von 4 Stunden erfolgt, und anschließend einer zweiten Wärmebehandlung unterzogen, welche bei einer Temperatur von 985 °C und über einer Zeitraum von 1 Stunde erfolgt.

Es wird eine erfindungsgemäße Spinell-Glaskeramik erhalten, die eine maximale Biegebruchfestigkeit, gemessen nach DIN EN ISO 6872, von 433 MPa aufweist. Durch Untersuchung der hergestellten Glaskeramik mittels Röntgendiffraktometrie und TEM wurde festgestellt, dass diese eine Spinell-Phase (bzw. Spinell-Kristallphase) und eine Lanthanphosphat-Kristallphase aber keine Hochquarzmischkristallphase enthält.

## Patentansprüche

1. Spinell-Glaskeramik aus einer Zusammensetzung mit den folgenden Komponenten:
25 bis 50 Gew.-% SiO₂,
10 bis 35 Gew.-% Al₂O₃,
1 bis 15 Gew.-% MgO,
1 bis 15 Gew.-% P₂O₅,
1 bis 25 Gew.-% ZrO₂ und/oder TiO₂,
0,1 bis 20 Gew.-% La₂O₃,
0 bis 10 Gew.-% B₂O₃, sowie
0 bis 15 Gew.-% Zusatzstoffe,
wobei sich die Anteile der Komponenten zu 100 Gew.-% ergänzen,
wobei die Spinell-Glaskeramik eine Glaskeramik ist, die mindestens eine Spinell-Phase enthält,
wobei die Spinell-Glaskeramik eine Lanthanphosphat-Kristallphase aufweist, und
wobei die Spinell-Glaskeramik keine Hochquarzmischkristallphase enthält.

2. Spinell-Glaskeramik nach dem vorhergehenden Anspruch, aus einer Zusammensetzung mit den folgenden Komponenten:
30 bis 45 Gew.-%, bevorzugt 35 bis 40 Gew.-% SiO₂,
15 bis 30 Gew.-%, bevorzugt 20 bis 25 Gew.-% Al₂O₃,
3 bis 10 Gew.-%, bevorzugt 5 bis 8 Gew.-% MgO,
2 bis 10 Gew.-%, bevorzugt 3 bis 8 Gew.-% P₂O₅,
8 bis 20 Gew.-%, bevorzugt 12 bis 18 Gew.-% ZrO₂ und/oder TiO₂,
0,1 bis 15 Gew.-%, bevorzugt 8 bis 12 Gew.-% La₂O₃,
0,1 bis 3,0 Gew.-%, bevorzugt 1,0 bis 2,0 Gew.-% B₂O₃, sowie
0,1 bis 10,0 Gew.-%, bevorzugt 1,0 bis 8,0 Gew.-% Zusatzstoffe.

3. Spinell-Glaskeramik nach einem der vorhergehenden Ansprüche, **dadurch gekennzeichnet, dass** die Spinell-Glaskeramik Spinell als Hauptkristallphase aufweist, wobei vorzugsweise der Anteil der Spinell-Hauptkristallphase an allen in der Spinell-Glaskeramik vorliegenden Kristallphasen mindestens 30 Gew.-%, besonders bevorzugt mindestens 50 Gew.-%, ganz besonders bevorzugt mindestens 70 Gew.-%, beträgt.

4. Spinell -Glaskeramik nach einem der vorhergehenden Ansprüche, **dadurch gekennzeichnet, dass** die Zusatzstoffe ausgewählt sind aus der Gruppe bestehend aus Fluor, Natriumoxid, Bariumoxid, Strontiumoxid, Zinkoxid, Calciumoxid, Yttriumoxid, Nioboxid, Tantaloxid, Lanthanoxid, Keimbildnern, insbesondere Zinnoxid und/oder Edelmetalle, Fluoreszenzmitteln, Farbstoffen, Hilfsstoffen, sowie Mischungen hiervon.

5. Spinell -Glaskeramik nach dem vorhergehenden Anspruch, **dadurch gekennzeichnet, dass** die Fluoreszenzmittel ausgewählt sind aus der Gruppe bestehend aus Oxiden von Neodym, Praseodym, Samarium, Europium, Terbium, Dysprosium, Holmium, Erbium, sowie Mischungen hiervon.

6. Spinell -Glaskeramik nach einem der Ansprüche 4 oder 5, **dadurch gekennzeichnet, dass** die Farbstoffe ausgewählt sind aus der Gruppe bestehend aus
- glasfärbenden Oxiden, bevorzugt Oxiden von Eisen, Titan, Cer, Kupfer, Chrom, Cobalt, Nickel, Mangan, Selen, Silber, Indium, Gold und Oxiden von Seltenerdmetallen
- Farbkörpern, bevorzugt dotierten Spinellen, sowie
- Mischungen hiervon.

7. Spinell -Glaskeramik nach einem der vorhergehenden Ansprüche, **dadurch gekennzeichnet, dass** die Spinell -Glaskeramik eine Biegebruchfestigkeit, gemessen nach DIN EN ISO 6872, von mehr als 400 MPa, vorzugsweise von mehr als 500 MPA, aufweist.

8. Verfahren zur Herstellung einer Spinell -Glaskeramik gemäß einem der vorhergehenden Ansprüche bei dem
a) ein Ausgangsglas hergestellt wird, das die Komponenten der Spinell -Glaskeramik enthält,
b) das Ausgangsglas mindestens einer ersten Wärmebehandlung und anschließend mindestens einer zweiten Wärmebehandlung unterzogen wird.

9. Verfahren nach dem vorhergehenden Anspruch, **dadurch gekennzeichnet, dass** die mindestens eine erste Wärmebehandlung bei einer Temperatur von 700 °C bis 950 °C, vorzugsweise von 800 °C bis 900 °C, und/oder über einen Zeitraum von 15 min bis 360 min, vorzugsweise von 120 min bis 240 min, erfolgt.

10. Verfahren nach einem der Ansprüche 8 oder 9, **dadurch gekennzeichnet, dass** die mindestens eine zweite Wärmebehandlung bei einer Temperatur von 900 °C bis 1200 °C, vorzugsweise von 950 °C bis 1000 °C, und/oder über einen Zeitraum von 5 min bis 200 min, vorzugsweise von 60 min bis 120 min, erfolgt.

11. Verwendung einer Spinell-Glaskeramik gemäß einem der Ansprüche 1 bis 7 als Dentalmaterial, als Komponente eines Dentalmaterials oder als hochfestes Substrat für Bauteile, insbesondere elektronische Bauteile.

12. Geformtes Dentalprodukt enthaltend eine Spinell-Glaskeramik gemäß einem der Ansprüche 1 bis 7, insbesondere in Form eines Inlays, eines Onlays, einer Brücke, eines Stiftaufbaus, einer Verblendung, einer Krone oder einer Teilkrone.

## Claims

1. Spinel glass-ceramic having a composition with the following components:
25 to 50% by weight SiO₂,
10 to 35% by weight Al₂O₃,
1 to 15% by weight MgO,
1 to 15% by weight P₂O₅,
1 to 25% by weight ZrO₂ and/or TiO₂,
0.1 to 20% by weight La₂O₃,
0 to 10% by weight B₂O_{3,} and
0 to 15% by weight additives,
the proportions of the components adding up to 100% by weight,
wherein the spinel glass-ceramic is a glass-ceramic containing at least one spinel phase,
wherein the spinel glass-ceramic comprises a lanthanum phosphate crystal phase, and
the spinel glass-ceramic not containing any high quartz solid solution phase.

2. The spinel glass-ceramic according to the preceding claim, having a composition with the following components:
30 to 45% by weight, preferably 35 to 40% by weight SiO₂,
15 to 30% by weight, preferably 20 to 25% by weight Al₂O₃,
3 to 10% by weight, preferably 5 to 8% by weight MgO,
2 to 10% by weight, preferably 3 to 8% by weight P₂O₅,
8 to 20% by weight, preferably 12 to 18% by weight ZrO₂ and/or TiO₂,
0.1 to 15% by weight, preferably 8 to 12% by weight La₂O₃,
0.1 to 3.0% by weight, preferably 1.0 to 2.0% by weight B₂O₃, and
0.1 to 10.0% by weight, preferably 1.0 to 8.0% by weight additives.

3. Spinel glass-ceramic according to any one of the preceding claims, **characterized in that** the spinel glass-ceramic has spinel as the main crystal phase, wherein the proportion of the spinel main crystal phase in all crystal phases present in the spinel glass ceramic is preferably at least 30% by weight, particularly preferably at least 50% by weight, very particularly preferably at least 70% by weight.

4. Spinel glass-ceramic according to any one of the preceding claims, **characterized in that** the additives are selected from the group consisting of fluorine, sodium oxide, barium oxide, strontium oxide, zinc oxide, calcium oxide, yttrium oxide, niobium oxide, tantalum oxide, lanthanum oxide, nucleating agents, in particular tin oxide and/or precious metals, fluorescent agents, dyes, auxiliaries, and mixtures thereof.

5. Spinel glass-ceramic according to the preceding claim, **characterized in that** the fluorescent agents are selected from the group consisting of oxides of neodymium, praseodymium, samarium, europium, terbium, dysprosium, holmium, erbium, and mixtures thereof.

6. Spinel glass-ceramic according to any one of claims 4 or 5, **characterized in that** the dyes are selected from the group consisting of
- glass-coloring oxides, preferably oxides of iron, titanium, cerium, copper, chromium, cobalt, nickel, manganese, selenium, silver, indium, gold and oxides of rare earth metals
- color bodies, preferably doped spinels, and
- mixtures thereof.

7. Spinel glass-ceramic according to any one of the preceding claims, **characterized in that** the spinel glass-ceramic has a flexural strength, measured according to DIN EN ISO 6872, of more than 400 MPa, preferably more than 500 MPa.

8. Method for producing a spinel glass-ceramic according to any one of the preceding claims in which
a) a starting glass containing the components of the spinel glass-ceramic is produced,
b) the starting glass is subjected to at least one first heat treatment and then to at least one second heat treatment.

9. Method according to the preceding claim, **characterized in that** the at least one first heat treatment takes place at a temperature of 700°C to 950°C, preferably from 800°C to 900°C, and/or over a period of 15 min to 360 min, preferably 120 min to 240 min.

10. Method according to any one of claims 8 or 9, **characterized in that** the at least one second heat treatment takes place at a temperature of 900°C to 1200°C, preferably from 950°C to 1000°C, and/or over a period of 5 min to 200 min, preferably of 60 min to 120 min.

11. Use of a spinel glass-ceramic according to any one of claims 1 to 7 as a dental material, as a component of a dental material or as a high-strength substrate for components, in particular electronic components.

12. Shaped dental product containing a spinel glass-ceramic according to any one of claims 1 to 7, in particular in the form of an inlay, an onlay, a bridge, a pin structure, a facing, a crown or a partial crown.

## Revendications

1. Vitrocéramique à base de spinelle, comprenant les composants ci-dessous :
entre 25 et 50 % en poids de SiO₂,
entre 10 et 35 % en poids d'Al₂O₃,
entre 1 et 15 % en poids de MgO,
entre 1 et 15 % en poids de P₂O₅,
entre 1 et 25 % en poids de ZrO₂ et/ou de TiO₂,
entre 0,1 et 20 % en poids de La₂O₃,
entre 0 et 10 % en poids de B₂O₃, ainsi que
entre 0 et 15 % en poids d'additifs,
dans laquelle les proportions des composants se complètent pour obtenir 100 % en poids,
dans laquelle la vitrocéramique à base de spinelle est une vitrocéramique contenant au moins une phase spinelle,
dans laquelle la vitrocéramique à base de spinelle présente une phase cristalline de phosphate de lanthane, et
dans laquelle la vitrocéramique à base de spinelle ne contient pas de phase cristalline mixte à quartz bêta.

2. Vitrocéramique à base de spinelle selon la revendication précédente, comprenant les composants ci-dessous :
entre 30 et 45 % en poids, de manière préférée entre 35 et 40 % en poids de SiO₂,
entre 15 et 30 % en poids, de manière préférée entre 20 et 25 % en poids d'Al₂O₃,
entre 3 et 10 % en poids, de manière préférée entre 5 et 8 % en poids de MgO,
entre 2 et 10 % en poids, de manière préférée entre 3 et 8 % de P₂O₅,
entre 8 et 20 % en poids, de manière préférée entre 12 et 18 % en poids de ZrO₂ et/ou de TiO₂,
entre 0,1 et 15 % en poids, de manière préférée entre 8 et 12 % en poids de La₂O₃,
entre 0,1 et 3,0 % en poids, de manière préférée entre 1,0 et 2,0 % en poids de 'B₂O₃,ainsi que
entre 0,1 et 10,0 % en poids, de manière préférée entre 1,0 et 8,0 % en poids d'additifs.

3. Vitrocéramique à base de spinelle selon l'une quelconque des revendications précédentes, **caractérisée en ce que** la vitrocéramique à base de spinelle présente du spinelle en tant que phase cristalline principale, dans laquelle la proportion de la phase cristalline principale de spinelle par rapport à toutes les phases cristallines présentes dans la vitrocéramique à base de spinelle est de manière préférée d'au moins 30 % en poids, de manière particulièrement préférée d'au moins 50 % en poids, et de la manière la plus particulièrement préférée d'au moins 70 % en poids.

4. Vitrocéramique à base de spinelle selon l'une quelconque des revendications précédentes, **caractérisée en ce que** les additifs sont choisis dans le groupe constitué du fluor, de l'oxyde de sodium, de l'oxyde de baryum, de l'oxyde de strontium, de l'oxyde de zinc, de l'oxyde de calcium, de l'oxyde d'yttrium, de l'oxyde de niobium, de l'oxyde de tantale, de l'oxyde de lanthane, d'agents de nucléation, en particulier de l'oxyde d'étain et/ou de métaux précieux, d'agents de fluorescence, de colorants, de substances auxiliaires, et de leurs mélanges.

5. Vitrocéramique à base de spinelle selon la revendication précédente, **caractérisée en ce que** les agents de fluorescence sont choisis parmi le groupe constitué des oxydes de néodyme, de praséodyme, de samarium, d'europium, de terbium, de dysprosium, d'holmium, d'erbium, et de leurs mélanges.

6. Vitrocéramique à base de spinelle selon la revendication 4 ou 5, **caractérisée en ce que** les colorants sont choisis dans le groupe constitué des
- oxydes colorants pour verre, de manière préférée des oxydes de fer, de titane, de cérium, de cuivre, de chrome, de cobalt, de nickel, de manganèse, de sélénium, d'argent, d'indium, d'or et des oxydes de terres rares
- pigments, de manière préférée des spinelles dopés, ainsi que des
- mélanges de ceux-ci.

7. Vitrocéramique à base de spinelle selon l'une quelconque des revendications précédentes, **caractérisée en ce que** la vitrocéramique à base de spinelle présente une résistance à la flexion, mesurée selon la norme DIN EN ISO 6872, supérieure à 400 MPa, de manière préférée supérieure à 500 MPa.

8. Procédé de fabrication d'une vitrocéramique à base de spinelle selon l'une quelconque des revendications précédentes, dans lequel
a) un verre de départ contenant les composants de la vitrocéramique à base de spinelle est fourni,
b) le verre de départ est soumis à au moins un premier traitement thermique, puis à au moins un second traitement thermique.

9. Procédé selon la revendication précédente, **caractérisé en ce que** le au moins un premier traitement thermique intervient à une température comprise entre 700 °C et 950 °C, de manière préférée comprise entre 800 °C et 900 °C, et/ou sur une période comprise entre 15 min et 360 min, de manière préférée comprise entre 120 min et 240 min.

10. Procédé selon l'une quelconque des revendications 8 ou 9, **caractérisé en ce que** le au moins un second traitement thermique intervient à une température comprise entre 900 °C et 1200 °C, de manière préférée comprise entre 950 °C et 1000 °C, et/ou sur une période comprise entre 5 min et 200 min, de manière préférée comprise entre 60 min et 120 min.

11. Utilisation d'une vitrocéramique à base de spinelle selon l'une quelconque des revendications 1 à 7 comme matériau dentaire, comme composant d'un matériau dentaire ou comme substrat à haute résistance pour des composants, en particulier des composants électroniques.

12. Produit dentaire façonné comprenant une vitrocéramique à base de spinelle selon l'une quelconque des revendications 1 à 7, en particulier sous la forme d'un inlay, d'un onlay, d'un bridge, d'un pivot, d'une facette, d'une couronne ou d'une couronne partielle.
